# EUROPEAN PATENT APPLICATION

(11) **EP 2 993 197 A1**
(43) Date of publication of application: **09.03.2016**
(21) Application number: 15185967.5
(22) Date of filing: 29.11.2007
(51) Int. Cl.: C08G 65/32, C08G 65/325, C07D 471/06, C09D 11/00, C09B 3/28, C09B 5/62, C09B 29/40

(54) **PHTHALOCYANINE COLORANTS AND THEIR USE AS FLUORESCENT SECURITY TAGGANTS**

(30) Priority: 29.11.2006 US 867660 P
(62) Divisional of application: 07864894.6
(71) Applicant: Sun Chemical Corporation, Parsippany, NJ 07054 (US)
(72) Inventor: SCHWARTZ, Russell, Cincinnati, OH Ohio 45232 (US); DERUSSY, Don, Mason, OH Ohio 45040 (US); GLOSTER, Dan, San Diego, CA California 92116 (US); POSTLE, Steve, Glen Rock, NJ New Jersey 07452 (US); VIP, Rakesh, Durham, CT Connecticut 06422 (US); COOK, Ewell, Middletown, CT Connecticut 06457 (US); ROMANOVA, Tanya, Loveland, OH Ohio 45140 (US)
(74) Representative: Fletcher, Matthew James Edwin

(57) **Abstract**

Certain phthalocyanine colorants are disclosed along with method of preparation thereof. A method of using these colorants as fluorescent security taggants is also disclosed.

## Description

### FIELD OF THE INVENTION

The invention relates to specific poly(oxyalkylene) colorants and methods of preparation thereof. The invention also relates to the use of these colorants as fluorescent security taggants.

Poly(oxyalkylene) polymeric colourants have been utilized to permanently color myriad substrates, including thermoplastic resins, such as disclosed in U.S. Pat. Nos. 4,284,729, 4,507,407, and 4,751,254; polyurethane foams, such as disclosed in U.S. Pat. Nos. 4,846,846; aqueous and non-aqueous liquids, such as disclosed in U.S. Pat. No. 4,871,371; and have been used as fugitive tints for textiles and threads, such as disclosed in U.S. Pat. No. 4,167,510. Such colorants provide effective and stable colorations to such materials, are easily handled, and exhibit desirable migratory properties within certain substrates.

3,4,9,10-Perylene tetracarboxylic acid bisimide based colorants are well known in industry and academia due to their favorable combination of light fastness, weather and thermal stability, as well as migration resistance in a number of applications such as coatings and plastics and inks. For this reason, they have been used extensively in the especially demanding applications of the automotive industry. These materials are insoluble pigments.

Patents DE 1130099 and GB 967178 first disclosed the potential for using perylenes as fluorescent dyes with high fluorescence quantum yield and photostability. However, these materials were only slightly soluble in organic solvents. An improvement in solubility was demonstrated by Langhals, et al, in Heterocycles, 1995, 40, 477 and references cited therein. Langhals reported that certain solubilizing substituents attached at the imide nitrogen such as long chain secondary alkyl groups (swallow-tail substituents) and 2,5-di tert-butylbenzene can enhance the solubility of perylene bisimdes in common organic solvents. These perylene bisimide dyes exhibit intense yellow fluorescence in solution with a quantum yield near unity. Even with the above-enhanced solubilizing groups, however, the materials are only soluble in common organic solvents at low concentrations. More recent applications for perylene bisimide dyes include dye laser applications, n-type semiconductors, and electrophotography.

Poly(oxyalkylene) substituents attached to organic chromophors have been used to make various colorants with enhanced dispersability and/or solubility, as disclosed in US 4,141,684, US 5,149,800, US 5,177,200, US 5,240,464, US 5,270,363, US 5,591,833, US 5,766,268, US 5,935,272, US 5,973,064, US 5,998,621 and the references cited therein. Applications reported thus far do not include the use of poly(oxyalkylene)ated chromophoric materials, especially perylene based materials, as fluorescent security taggants.

### SUMMARY OF THE INVENTION

The present invention provides a perylene poly(oxyalkylene) bisamide compound having the following chemical formula **I:** 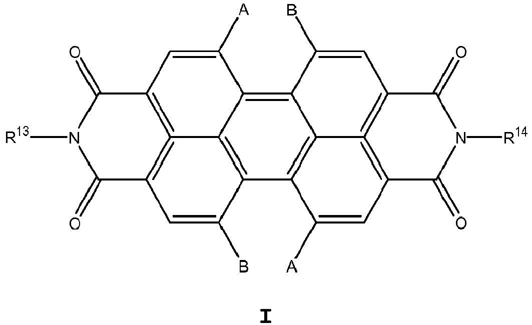 wherein each of A and B is independently selected from the group consisting of: H, OH, OR⁴, N(R³)₂, alkyl, F, CL, Br, I, NO₂, CN, ⁻NCS, ⁻SCN; R³ is selected from the group consisting of: alkyl, aralkyl, alkaryl, polyalkylene oxide; and each of R¹³ and R¹⁴ is independently a polyalkylene oxide.

The present invention also provides a perylene poly(oxyalkylene) tetraamide compound having the following chemical formula **II:** 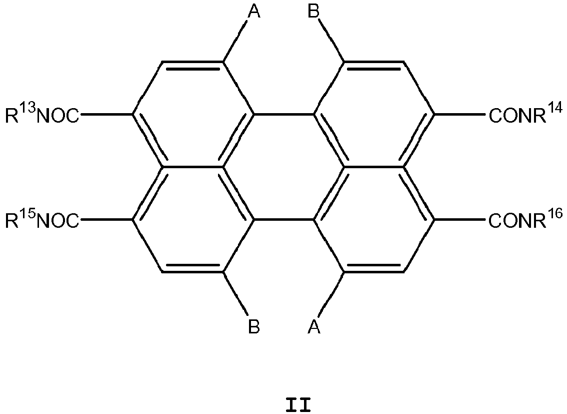 wherein each of A and B is independently selected from the group consisting of: H, OH, OR, N(R³)₂, alkyl, F, CL, Br, I, NO₂, CN, ⁻NCS, ⁻SCN; R³ is selected from the group consisting of: alkyl, aralkyl, alkaryl, polyalkylene oxide; and each of R¹³ , R¹⁴ , R¹⁵ and R¹⁶ is independently a polyalkylene oxide

The present invention further provides a triarylmethane compound having one of the following chemical formulae **III or IV:** 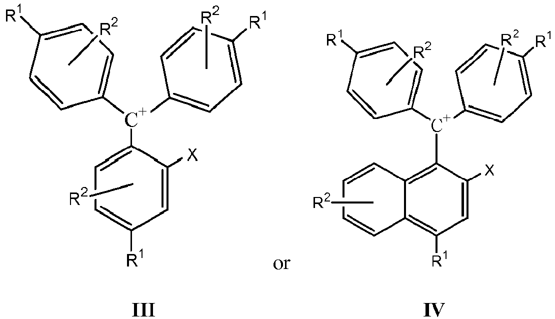 wherein X is H or CO₂⁻ or CO₂H [with a counterion Z⁻]; R¹ is selected from the group consisting of H, NR³₂, OR⁴, alkyl, aryl and substituted aryl; R² is selected from the group consisting of H, SO₃H, SO₃-(1/n)Mⁿ⁺, CO₂H and CO₂-(1/n)Mⁿ⁺; each of R³, R⁴ is independently selected from the group consisting of alkyl, aralkyl, alkaryl and polyalkylene oxide; Z is selected from the group consisting of halide, tosylate, brosylate, carboxylate, sulphate and phosphate; M is a cation; and n is integer from 1 to 4.

The present invention further provides a xanthene or thioxanthene compound having one of the following chemical formula **V:** 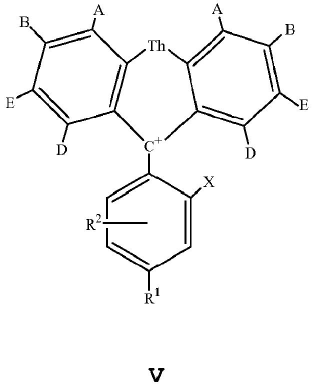 wherein Th is O or S; R¹ is selected from the group consisting of H, NR³₂, OR⁴, alkyl, aryl and substituted aryl; R² is selected from the group consisting of H, SO₃H, SO₃⁻(-/n)Mⁿ⁺, CO₂H and CO₂-(1/n)Mⁿ⁺; and each of A, B, E and D is independently selected from the group consisting of H, OH, OR⁴, NR³₂, F, CL, Br, I, NO₂, CN, ⁻NCS and ⁻SCN.

The present invention also provides a cyanine compound having the following chemical formula **VI:** 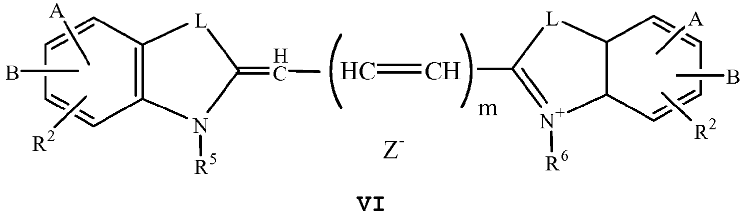 wherein L is selected from the group consisting of O, S and N-R⁷; A and B is independently selected from the group consisting of H, OH, OR⁴, NR³₂, F, CL, Br, I, NO₂, CN, ⁻NCS and ⁻SCN; R² is selected from the group consisting of H, SO₃H, SO₃-(1/n)Mⁿ⁺, CO₂H and CO₂- (1/n)Mⁿ⁺; m is integer from 0 to 3; Z is selected from the group consisting of halide, tosylate, brosylate, carboxylate, sulphate and phosphate; and each of R⁵, R⁶, R⁷ is independently selected from the group consisting of alkyl, carboxyalkyl, alkylsulfonate, alkylaryl and polyalkylene oxide.

The present invention further provides a diazopyrrole compound having the following chemical formula **VII:** 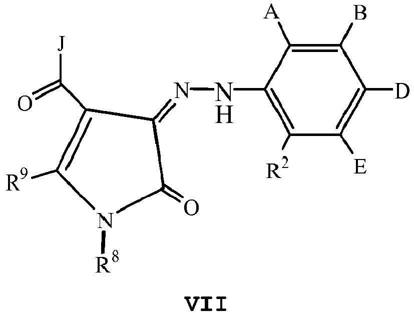 wherein each of A, B, E and D is independently selected from the group consisting of H, OH, OR⁴, N(R³)₂, F, CL, Br, I, NO₂, CN, -NCS, -SCN and substituted aryl; R² is selected from the group consisting of H, SO₃H, SO₃⁻(1/n)Mⁿ⁺, CO₂H and CO₂-(1/n)Mⁿ⁺; each of R³ and R⁴ is independently selected from the group consisting of alkyl, aralkyl, alkaryl and polyalkylene oxide; R⁸ is selected from the group consisting of H, alkyl, aryl, substituted aryl, aralkyl, alkaryl and imidino; R⁹ is selected from the group consisting of H, alkyl, ⁻N(R³)₂, ⁻OR⁴, aryl and substituted aryl; and J is NH-polyalkyene oxide or O-polyalkylene oxide.

Preferably, the diazopyrrole compound of chemical formula **VII** has the following chemical formula **XXV:** 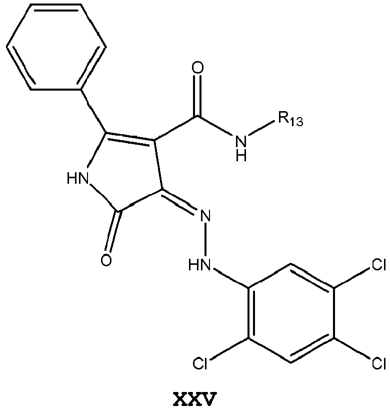 wherein R¹³ comprises (CₐH₂ₐO)ₘ(C_{b}H_{2b}O)ₙOCH₃, where a and b are different and either 2 or 3, m is at least 3, and n is 1 - 31.

The present invention also provides a phthalocyanine compound having the following chemical formula **IX:** 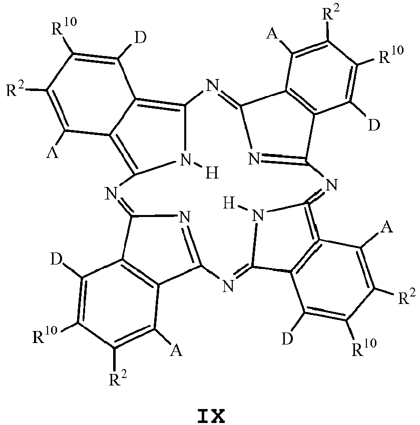 wherein each of A and D is independently selected from the group consisting of H, OH, OR⁴, N(R³)₂, F, CL, Br, I, NO₂, CN, ⁻NCS and ⁻SCN; R² is selected from the group consisting of H, SO₃H, SO₃⁻(1/n)Mⁿ⁺, CO₂H and CO₂-(1/n)Mⁿ⁺; and R¹⁰ is selected from the group consisting of R², A, N(R³)₂, OR⁴ and SR⁴.

The present invention further provides a phthalocyanine compound having the following chemical formula **X:** 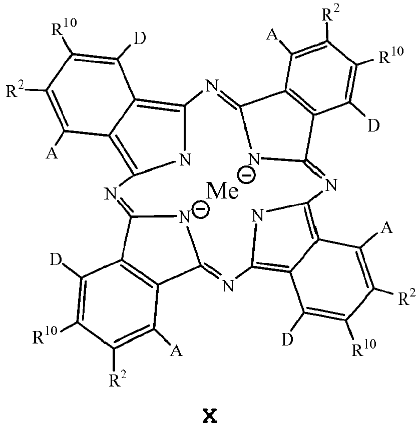 wherein Me is MXₙ(O)ₚ²⁺, M is a cation derived from a metallic or semimetallic element, X is a halide or alkoxide, O is an oxygen atom, n is an integer from 0 to 3, and p is an integer from 0 to 1, each of A and D is independently selected from the group consisting of H, OH, OR⁴, N(R³)₂, F, CL, Br, I, NO₂, CN, ⁻NCS and ⁻SCN; R² is selected from the group consisting of H, SO₃H, SO₃⁻(1/n)Mⁿ⁺, CO₂H and CO₂-(1/n) Mⁿ⁺; and R¹⁰ is selected from the group consisting of R², A, N(R³)₂, OR⁴ and SR⁴.

The present invention also provides a naphthalocyanine compound having the following chemical formula **XI:** 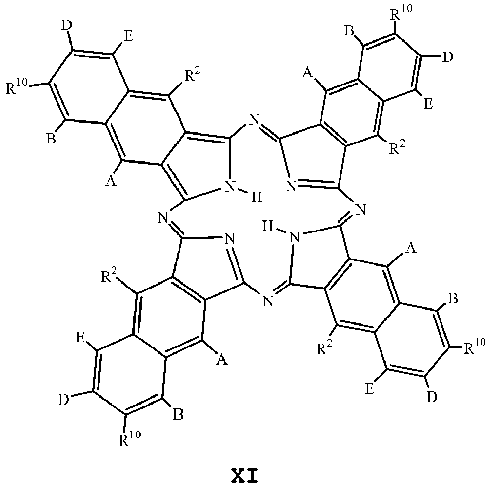 wherein each of A, D and E is independently selected from the group consisting of H, OH, OR⁴, NR³₂, F, CL, Br, I, NO₂, CN, ⁻NCS and ⁻SCN; R² is selected from the group consisting of H, SO₃H, SO₃⁻(1/n)Mⁿ⁺, CO₂H and CO₂-(1/n)Mⁿ⁺; and R¹⁰ is selected from the group consisting of R², A, N(R³)₂, OR⁴ and SR⁴.

The present invention further provides a naphthalocyanine compound having the following chemical formula **XII:** 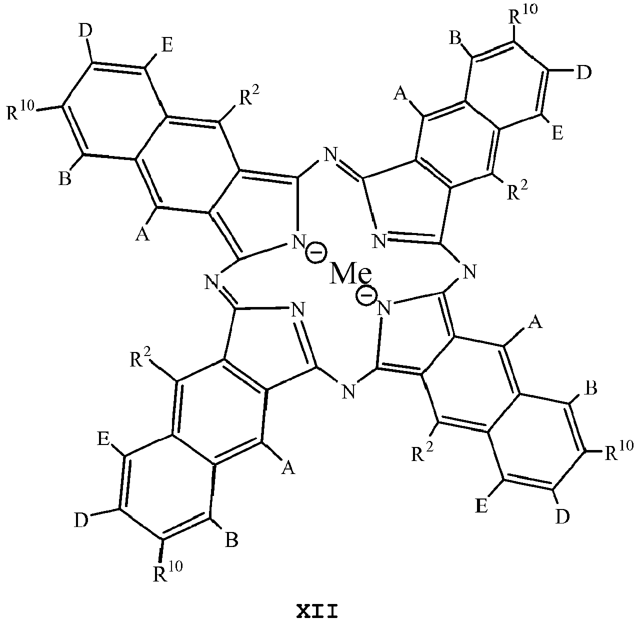 wherein Me is MXₙ(O)ₚ²⁺, M is a cation derived from a metallic or semimetallic element, X is a halide or alkoxide, O is an oxygen atom, n is an integer from 0 to 3, and p is an integer from 0 to 1, each of A, D and E is independently H, OH, OR⁴, NR³₂, F, CL, Br, I, NO₂, CN, ⁻NCS and SCN; R² is selected from the group consisting of H, SO₃H, SO₃⁻(1/n)Mⁿ⁺, CO₂H and CO₂-(1/n)Mⁿ⁺; and R¹⁰ is selected from the group consisting of R², A, N(R³)₂, OR⁴ and SR⁴.

The present invention also provides a coumarin or a carbostyril compound having the following chemical formula **XIII:** 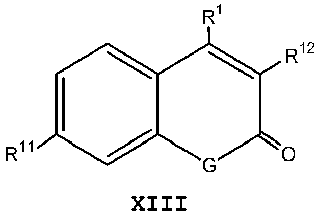 wherein R¹ is selected from the group consisting of H, N(R³)₂, OR⁴, alkyl, aryl and substituted aryl; R¹¹ is selected from the group consisting of: N(R³)₂,

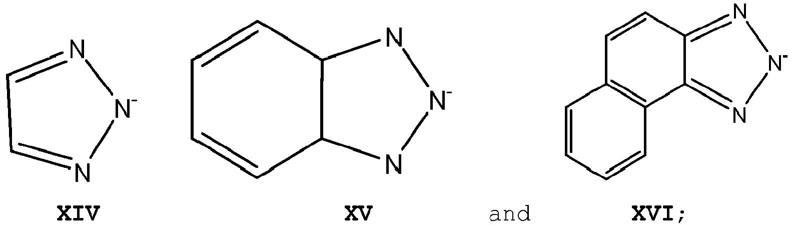

R¹² is or A; G is O or NR⁵; R⁵ is independently selected from the group consisting of alkyl, carboxyalkyl, alkylsulfonate, alkylaryl and polyalkylene oxide; and A is selected from the group consisting of H, OH, OR⁴, NR³₂, F, CL, Br, I, NO₂, CN, ⁻NCS and ⁻SCN.

The present invention further provides a dialkyl succinosuccinate (DMSS) compound having the following chemical formula **XVII:** 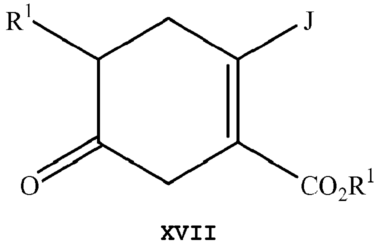 wherein R¹ is selected from the group consisting of H, NR³₂, OR⁴, alkyl, aryl and substituted aryl; and J is NH-polyalkyene oxide or O-polyalkylene oxide.

Preferably, the dialkyl succinosuccinate (DMSS) compound of chemical formula **XVII** has the following formula **XXVI:** 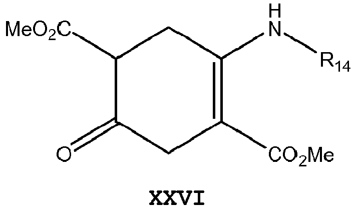 wherein R¹⁴ comprises (CₐH₂ₐO)ₘ(C_{b}H_{2b}O)ₙCH₃, where a and b are different and either 2 or 3, m is at least 3, and n is 1 - 31.

The present invention also provides a naphthaloperylene compound having the following chemical formula **XIX:** 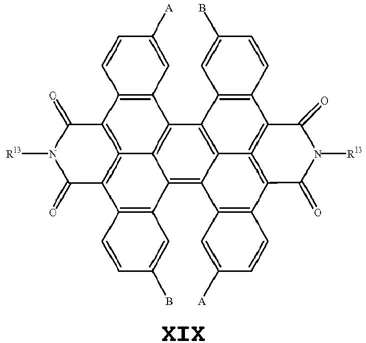 wherein each of A and B is independently H, OH, OR⁴, NR³₂, F, CL, Br, I, NO₂, CN, ⁻NCS and ⁻SCN; and R¹³ is polyalkylene oxide.

The present invention further provides a Lanthanide compound having the following chemical formula **XX:** 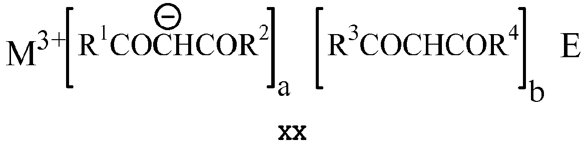 wherein M is a lanthanide; each of R1, R2 and R3 is independently selected from the group consisting of alkyl, alkaryl, aralkyl, cyano, haloalkyl, haloaryl, aryl, carboxy aryl, aryl sulfonate, heterocyclic and R⁴; R⁴ is selected from the group consisting of -NH [polyalkylene oxide], -O [polyalkylene oxide],
and 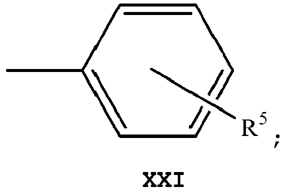 R⁵ is selected from the group consisting of -CONH [polyalkylene oxide], -CO₂ [polyalkylene oxide], -SO₃ [polyalkylene oxide] and -SO₂NO [polyalkylene oxide]; a is an integer from 0-2; b is an integer from 1-3; and E is 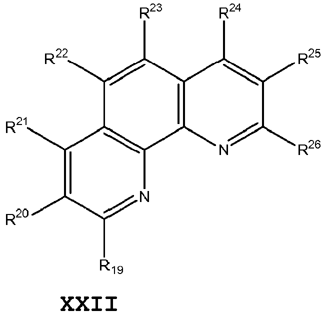 where R19 through R26 are each independently H, alkyl, alkoxy, halide, sulfonic acid.

The present invention also provides a Lanthanide compound having the following chemical formula **XXIII:** 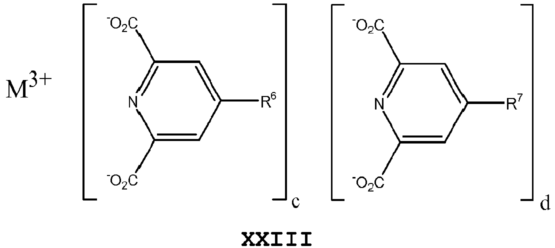 wherein M is a lanthanide; c is an integer from 0-2; d is an integer from 1-3; R⁶ is selected from the group consisting of H, alkyl, alkaryl, sulphonic acid, carboxylic acid, halide, alkoxy, sulphonamide, carboxamide and R⁷; and R⁷ is selected from the group consisting of CONH [polyalkylene oxide], CO₂ [polyalkylene oxide], SO₂NH [polyalkylene oxide] and SO₃ [polyalkylene oxide].

The present invention further provides a method of tagging an article with a fluorescent taggant comprising coating said article with a coating composition containing a compound having the following chemical formula **XXIV:** 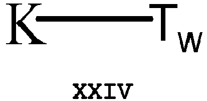 wherein K is a fluorescent moiety; T is a pendant group comprising a polyalkylene oxide residue; and w is a number greater than zero.

Other objects and advantages of the present invention will become apparent from the following description and appended claims.

### BRIEF DESCRIPTION OF THE DRAWINGS

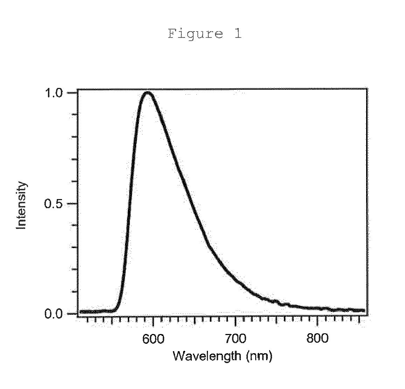

Figure 1 shows the spectrum for a printed sample of ink Formulation #1.

### DETAILED DESCRIPTION OF THE INVENTION

It has now been demonstrated that substituents such as poly(oxyalkylene) attached to pigment molecules or fluorescent compounds exhibit high fluorescence in ink formulations and are readily soluble in organic solvents are high concentrations. Accordingly, solutions of these materials, i.e. compounds of the present invention, exhibit high fluorescence in ink formulations such that they are useful for security taggant applications.

The pigment or fluorescent compounds used in preparing the compounds of the present invention include but are not limited to the group consisting of perylene, xanthene, thioxanthene, cyanine, diazopyrrole, phthalocyanine, naphthalocyanine, coumarin, carbostyril, dialkylsuccinosuccinate, naphthaloperylene and derivatives thereof.

The terms "coating composition" and "security applications" for the purpose of the present invention mean a composition to be applied to an underlying substrate of an article where such application is used to subsequently recognize such article.

Compounds of the present invention may be prepared by having substituents such as poly(oxyalkylene) attached via a nitrogen or oxygen moiety to a functional residue in the fluorescent colorant molecule. Examples of this, for the class of peylene colorants described in this invention, include either the imide nitrogen of perylene bisimides or the amide nitrogen of perylene tetraamides. These substituents are much more efficient at solubilizing a perylene molecule. These perylene poly(oxyalkylene) bisimides and tetraamides are readily soluble in common organic solvents in high concentrations. For other colorant types, the poly (alkylene oxide) moiety may be attached via an imide (for example, in dialkylsuccinosuccinate dyes) or an amide (for example, in azopyrrole dyes), as an ester, or as a sulfonamide, or via other functional residues known to those skilled in the art.

Perylene poly(oxyalkylene) bisimides are prepared by reacting purified perylene tetracarboxylic acid dianhydride with poly(oxyalkylene)amines, which are readily available from Huntsman Corp. The reaction is performed in a polar aprotic solvent such as n-methylpyrrolidinone with or without the presence of a Lewis acid catalyst such as zinc acetate dihydrate at a temperature preferably between 100 - 210 °C, most preferably between 150 - 200 °C.

Perylene poly(oxyalkylene) tetraamides are prepared in a two step process. The first step is reaction between a secondary amine and purified perylene tetracarboxylic acid dianhydride, giving a perylene bisamide, biscarboxylic acid. Preferred conditions are to perform this reaction in a polar solvent such an alcohol or water, most preferably water at a temperature between ambient to 100°C, most preferably 50°C. The second step is to react the perylene bisamide, biscarboxylic acid with a poly(oxyalkylene)amine. The reaction is performed in a polar aprotic solvent such as n-methylpyrrolidinone with or without the presence of a Lewis acid catalyst such as zinc acetate dihydrate at a temperature preferably between 100 - 210°C, most preferably between 150 - 200°C.

Compounds of the present invention, preferably, Perlyene poly(oxyalkylene) bisimides and Perlyene poly(oxyalkylene)tetra amides exhibit excellent light fastness for indoor applications. The excellent solubility of these materials in common ink vehicles allows for stable ink formulations that can be used in demanding applications such as in water-based ink jet inks, solvent-based ink jet inks, flexo inks, energy curable inks and Offset inks. The high fluorescence, light fastness and good solubility make these ideal fluorescent security taggants that can be formulated into jettable ink. Preferably, the compounds of the present invention are present in an amount from about 0.01 to about 20%, more preferably from about 0.1 to about 10% by weight of an ink or coating composition.

### Example 1 - a Perylene Poly(oxyalkylene) Bisimide Compound (Compound B)

In a 2000 ml 4-necked flask was di spersed 100.0 g 3,4,9,10-perylene tetracarboxylic acid dianhydride, 306.0 g of methoxypoly(oxyethylene/oxypropylene)-2-propylamine (MW = 600), 11.19 g zinc acetate dihydrate and 1000 ml n-methylpyrrolidinone. The mixture was heated to 200 °C, allowing the evolved water to distill out of the flask. Held at 200 °C for 15 hours, then cooled to room temperature and drowned into 10 liters water. The product was collected by filtration and washed with water until no n-methylpyrrolidinone was detected in the filtrate. The wet presscake was slurried into 6000 ml 5% aqueous sodium hydroxide and stirred at 80 °C for 1 hour, filtered and washed with water until the pH and conductivity of the filtrate was equivalent to that of the wash water, dried in an oven at 80°C. Perylene poly(oxyalkylene) bisimide (342.35g; 86 %) was isolated as a red rubbery solid. The product exhibited good solubility in common organic solvents such as acetone and methanol. In addition, it exhibited a significant fluorescence emission maximum near 553 nm when excited at 365 nm, 470 nm or 500 nm. When formulated into an ink-jet ink, printed on paper and exposed to visible or UV light, it showed excellent fluorescence.

### Example 2 - Preparation of a Perylene Poly(oxyalkylene) Tetraamide Compound

In a 2000 ml 4-necked flask, 3,4,9,10-perylene tetracarboxylic acid dianhydride presscake (150.0 g based on dry color) was dispersed into 1400 ml water. Diethylamine (112.05 g)was added and heated to 50°C and held for 3 hours, then cooled. The mixture was acidified with concentrated hydrochloric acid until the slurry did not bleed when spotted on filter paper. The product was filtered and washed with water until the pH and conductivity of the filtrate was equivalent to that of the wash water. Perylene bis diethylamide bis carboxylic acid presscake (610.55 g) was isolated with a moisture content of 27.58% (168.39 g dry, 82% yield).

In a 500 ml 4-necked flask, perylene bis diethylamide bis carboxylic acid presscake (10.0 g based on dry color) was dispersed into 100 ml n-methylpyrrolidinone. Methoxypoly(oxyethylene/oxypropylene)-2-propylamine(22.32 g; MW = 600) was added and heated to 200°C, allowing the evolved water to distill out of the flask. The temperature was held at 200 °C for 15 hours, then cooled to room temperature and drown into 1000 ml water. The product was collected by filtration and washed with water until no n-methylpyrrolidinone was detected in the filtrate. The collected product was then dried in an oven at 80°C. The isolated product (23.3 g; 74%) was a mixture of perylene bis diethylamide bis poly(oxyalkylene) amides as a red rubbery solid. The product exhibited good solubility in common organic solvents such as acetone and methanol. In addition, it exhibited a significant fluorescence emission maximum near 526 nm when excited at 365 nm, 470 nm or 500 nm. When formulated into an ink-jet ink, printed on paper and exposed to visible or UV light, it showed excellent fluorescence.

### Example 3 - Preparation of a Perylene Poly(oxyalkylene) Bisimide Compound

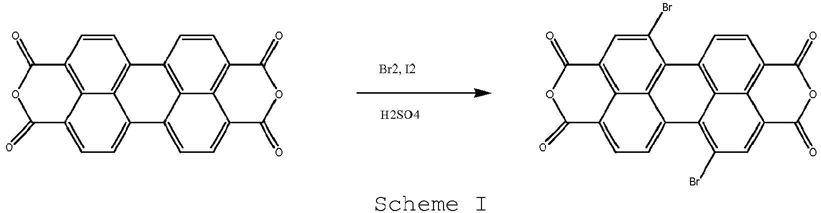

As outlined in scheme I above (described in German Patent DE 195 47 209 A1), the starting material (14.6g, 37.2mmol) was suspended in concentrated Sulfuric acid (221 g) and stirred overnight at room temperature. To the suspension was added I2 (350mg, 1.38 mmol), and the mixture heated to 85°C. Bromine (4.22ml, 82.6 mmol) was added dropwise over ∼30min. The mixture was allowed to stir for 20 hours at 85°C. After cooling, the excess bromine was removed by a stream of nitrogen. Water (35 ml) was added dropwise to the reaction mixture, and the suspension heated to 85°C for one hour. After cooling, the reaction mixture was filtered (M porosity glass frit) and washed repeatedly with water until the filtrate registered neutral to litmus. The red solid thus obtained was dried at 65-70C for 4 days. The yield was a red powder (18.75 g).

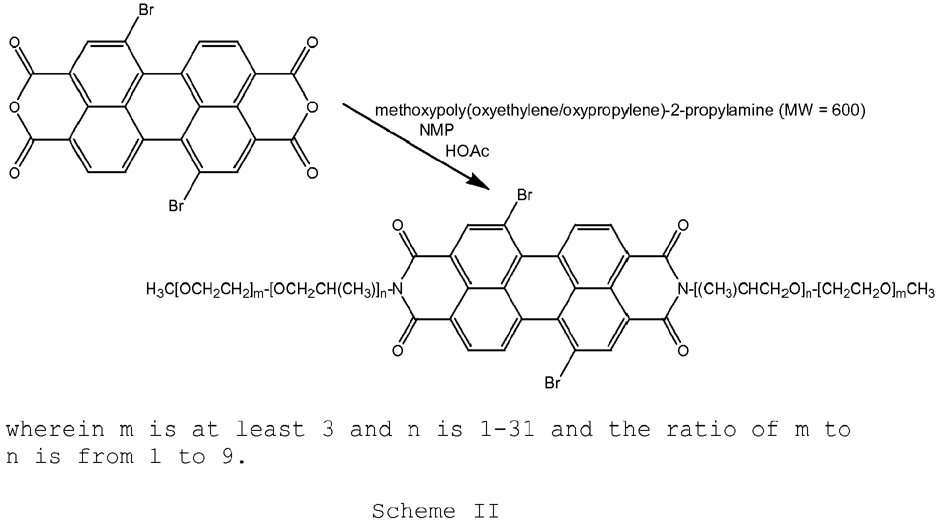

The dibromoperylene (5.0g, 9.1mmol) was suspended in 55ml N-methylpyrollidone containing 250µl of acetic acid, and heated to 95°C (bath temp). The methoxypoly(oxyethylene/oxypropylene)-2-propylamine (11g, ∼18.5mmol; MW = 600) was added in portions over 2 hours. Continued heating at 95C over the next 7 hours, during which time the reaction mixture gradually became homogenous. After stirring overnight at room temperature, the mixture was poured into 500 ml of water. An immediate tarry red material separated, which was isolated by decanting the supernatant liquid after standing for 1 hour. This reaction is described in Scheme II above. The tarry solid was dissolved in CH₂Cl₂, dried over Na₂SO₄, filtered and evaporated to yield approximately 11 g of a viscous red oil.

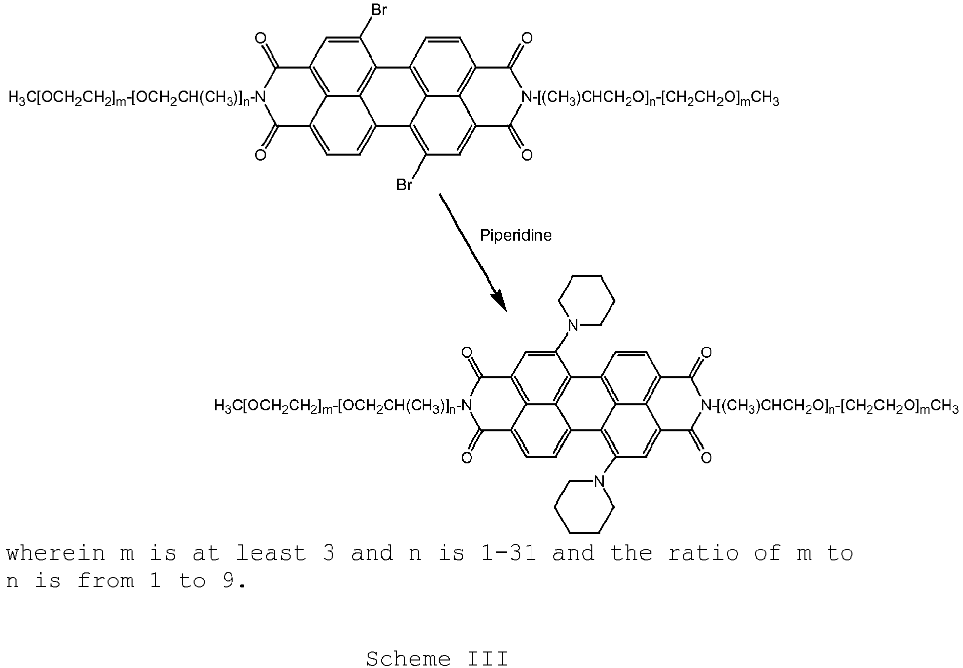

The dibromo compound (about 5 g) was added to piperidine (35 ml) and heated to 75°C under N₂ for 16 hours as indicated in Scheme III above. After cooling to room temperature, the mixture was evaporated, then evaporated again from toluene. The residue was eluted over silica with a gradient of CH₂Cl₂, followed by 1%, 2%, 3%, 4% EtOH in CH₂Cl₂ containing 0.2% Et3N. A blue-green band elutes first which appears as one spot on TLC. The yield was 1.9g of a blue-green oil, highly fluorescent, with an Emission max of 765 nm and an Excitation max of 693 nm in ethanol.

Similarly, pyrrolidine can be substituted for piperidine in the final reaction step to yield the bis-pyrrolidinyl product.

### Example 4 - Preparation of a DMSS Compound (Compound C):

Dimethyl 2,5-dioxo-1,4-cyclohexane dicarboxylate (8.94 g; 0.0392 mol)was dispersed into methoxypoly(oxyethylene/oxypropylene)-2-propylamine (80 g; 0.0392 mol; MW = 2000) in a 500 ml 4-necked flask. Five drops of concentrated hydrochloric acid was added to the dispersion which was then heated to 120°C while passing a slow stream of nitrogen over the reaction mixture. Water was then distilled out of the flask during the heat-up period and while at 120°C. After 2.5 hours, residual water was removed under vacuum. The cooled product shown below in chemical formula XVIII was isolated as an amber oil was soluble in common solvents such as acetone and exhibited fluorescent characteristics. 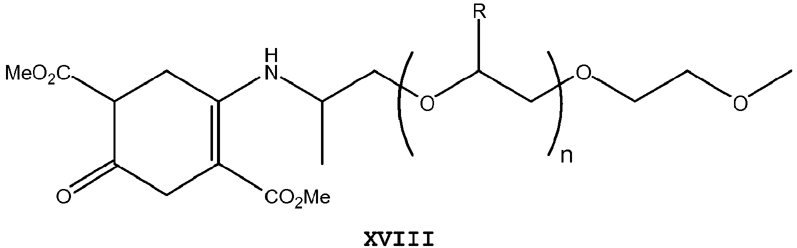 wherein R is 95% CH₃ and 5% H and n is about 35.

The final product of the DMSS compound (Compound C; about 1 mg) was dissolved separately into each of methanol (100 ml MeOH) and methyl ethyl ketone (100 ml MEK). The fluorescence was taken at excitation of 470 nm, excitation slit of 15.0 nm, emission slit of 15.0 nm with measurements from 200-900 nm @ 250 nm/minute. It was also determined that there was an excitation maximum at 345 nm so a second emission was determined using the same conditions but with an excitation wavelength of 345 nm. The fluorescence measurements are described in Table 1 below.

**Table 1**

| **Sample** | **@ 345 nm excitation** | | **@ 470 nm excitation** | |
|---|---|---|---|---|
| | **Max Wavelength** | **Intensity** | **Max Wavelength** | **Intensity** |
| Compound C in MEK | 441.08 | 360.24 | 520.55 | 257.45 |
| Compound C in MeOH | 445.25 | 365.54 | 524.04 | 119.02 |
| MEK Blank | | | 528.43 | 16.70 |
| MeOH Blank | 432.15 | 18.08 | | |

It is clear from the results shown above the Compound C has a significant fluorescence when excited at 345 nm and 470 nm.

### Example 5 - Preparation of an Azopyrrole Compound (Compound A) :

2-Phenyl-3-ethoxycarbonyl-4-(2,4,5-trichlorophenylhydrazonyl)-1H-5-pyrrolinone (4.00 g; 0.00912 mol) made analogously as described in Example 7 of US Patent No. 6,965,019 was mixed with methoxypoly(oxyethylene/oxypropylene)-2-propylamine (5.47 g; 0.00912 mol; MW = 600) and 1-methyl-2-pyrrolinidinone (NMP; 1 ml) in a 250 ml 4-necked flask. The mixture was heated to 200°C and held for 3.5 hours, at which time the NMP was removed under reduced pressure. The product was isolated upon cooling as a dark oil which dissolved into common solvents such as acetone and exhibited fluorescent characteristics. The product has the following chemical formula **VIII:** 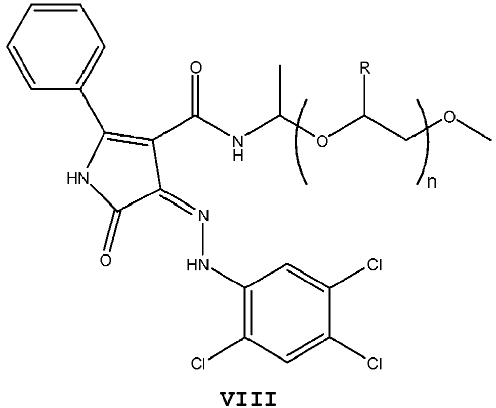 wherein R is 90% CH3 and 10% H and n an integer from about 9 to 10.

The final product of the azopyrrole compound (Compound A; about 1 mg) was dissolved separately into each of methanol (100 ml MeOH) and methyl ethyl ketone (100 ml MEK). The fluorescence was taken at excitation of 470 nm, excitation slit of 15.0 nm, emission slit of 15.0 nm with measurements from 200-900 nm @ 250 nm/minute. It was also determined that there was an excitation maximum at 345 nm so a second emission was determined using the same conditions but with an excitation wavelength of 345 nm. For a drawdown of the azopyrrole compound, a piece was cut and run using the paper attachment for the Fluorescence Spectrophotomoter using the previous conditions at 470 nm. A blank paper was run as well along with blanks of MEK and MeOH at each excitation. The fluorescence measurements are described in Table 2 below.

**Table 2**

| **Sample** | **@ 345 nm excitation** | | **@ 470 nm excitation** | |
|---|---|---|---|---|
| | **Max Wavelength** | **Intensity** | **Max Wavelength** | **Intensity** |
| Compound A in MEK | 428.96 | 956.59 | 524.3 | 147.73 |
| Compound A in MeOH | 424.97 | 158.4 | 525.03 | 112.06 |
| Compound A Drawdown | | | 526.17 | 111.52 |
| MEK Blank | | | 528.43 | 16.70 |
| MeOH Blank | 432.15 | 18.08 | | |
| Drawdown Blank | | | 520.46 | 57.13 |

It is clear from the results shown above the Compound A has a significant fluorescence when excited at 345 nm and 470 nm. However, Compound A has much stronger fluorescence emission when excited at 345 nm.

### Example 6 - Preparation of Ink Formulation #1 Containing the Azopyrrole Compound Prepared in Example 5

A continuous inkjet (CIJ) ink was prepared by using the material listed in Table 3 below. First, the Azopyrrole compound prepared in Example 5 (Compound A) was added to half of the total MEK (methylethylketone) volume followed by stirring until a solution was obtained. Subsequently the resin VMCC (Dow Chemical) was introduced into the mixture followed by further mixing to obtain a solution, followed by the addition of potassium thiocyanate and more mixing. A second solution was obtained by addition of the black colorant Microlith CK (Ciba) to the remainder of the MEK solvent and mixing by a high shear mixer at 10000 rpm for 40 minutes. The two mixtures were then combined, mixed and filtered through a 1 micron filter to obtain the finished ink.

**Table-3**

| **Components** | **% w/w** |
|---|---|
| | |
| MEK | 90.7 |
| Microlith CK | 0.5 |
| Compound A | 1.8 |
| Resin VMCC | 6.3 |
| KSCN | 0.7 |

The viscosity and conductivity of this ink were measured by standard methods and they were determined to be within the acceptable range as recommended for typical CIJ printers. Test printing was carried out using a Videojet Excel 2000 CIJ printer on a variety of substrates. The luminescence of printed samples were characterized by using an Ocean Optic H4000 spectrometer using a bifurcated probe, a halogen light source or a 488 nm Argon laser and appropriate optical filters. For solution samples, a Cary Eclipse spectrometer was used and a triangular cell for front face emission was utilized. The spectral information for a number of samples is indicated in Table 4. Figure 1 provides the spectrum for a printed sample of ink Formulation #1.

**Table 4**

| Material | Emission peak (nm) |
|---|---|
| Ink Formulation #1 | 543 |
| Ink Formulation #2 (see Example 7 below) | 570 |
| Ink Formulation #3 (see Example 8 below) | 574 |

### Example 7 - Preparation of Ink Formulation #3 Containing the Perylene Poly(oxyalkelene) Compound Prepared in Example 1

An additional CIJ ink was prepared by using the perylene poly(oxyalkelene) compound prepared in Example 1 (compound B) in a process similar to that of Example 6 based on the material indicated in Table 5.

**Table 5**

| **Components** | **% w/w** |
|---|---|
| | |
| MEK | 88.5 |
| Microlith CK | 3.6 |
| Compound B | 0.9 |
| Resin VMCC | 6.3 |
| KSCN | 0.7 |

### Example 8 - Preparation of Ink Formulation #2 Containing the DMSS Compound Prepared in Example 4

A different CIJ ink was prepared by using the DMSS compound prepared in Example 4 (Compound C) in a process similar to that of Examples 6 and 7 based on the material indicated in Table 4.

**Table 4**

| **Components** | **% w/w** |
|---|---|
| | |
| MEK | 90.7 |
| Microlith CK | 0.5 |
| Compound C | 1.8 |
| Resin VMCC | 6.3 |
| KSCN | 0.7 |

The invention has been described in terms of preferred embodiments thereof, but is more broadly applicable as will be understood by those skilled in the art. The scope of the invention is only limited by the following claims.

### Further aspects of the invention

Further aspects of the invention are described in the following numbered clauses:
1. A perylene poly(oxyalkylene) bisimide compound having the following chemical formula I: 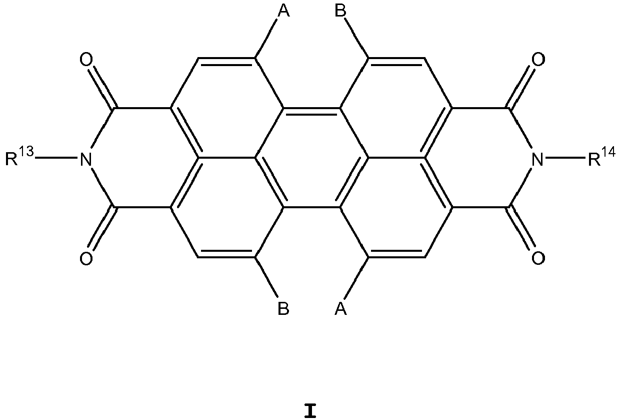 wherein each of A and B is independently selected from the group consisting of: H, OH, OR⁴, N(R³)₂, alkyl, F, CL, Br, I, NO₂, CN, -NCS, -SCN; R³ is selected from the group consisting of: alkyl, aralkyl, alkaryl, polyalkylene oxide; and each of R¹³ and R¹⁴ is independently a polyalkylene oxide.
2.The perylene compound of clause 1, wherein each of A and B is H; and each of R¹³ and R¹⁴ comprises (CₐH₂ₐO)ₘ(C_{b}H_{2b}O)ₙCH₃, where a and b are different and either 2 or 3, m is at least 3, and n is 1 - 31.
3. An ink or a coating composition comprising the perylene compound of clause 1.
4.The composition of clause 3, wherein said compound is present in an amount from about 0.01 to about 20% by weight.
5. The composition of clause 3 being a water-based ink jet ink.
6.The composition of clause 3 being a solvent based ink jet ink.
7. The composition of clause 3 being a flexo ink.
8. The composition of clause 3 being an energy curable ink.
9. The composition of clause 3 being an offset ink.
10.The composition of clause 3 being a coating composition for security applications.
11. An article coated with the composition of clause 3.
12. A method of tagging an article with a fluorescent taggant comprising coating said article with the coating composition of clause 3.
13.A perylene poly(oxyalkylene) tetraamide compound having the following chemical formula II: 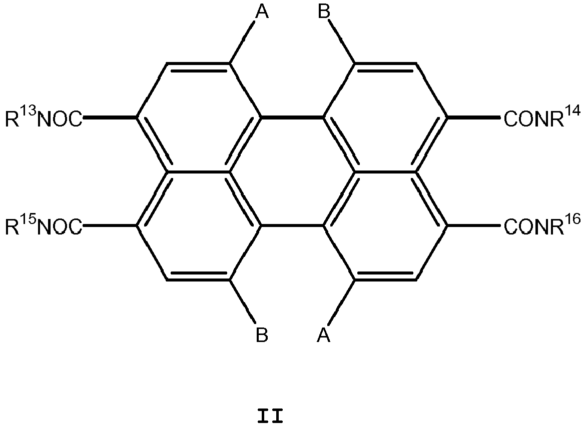 wherein each of A and B is independently selected from the group consisting of: H, OH, OR⁴, N(R³)₂, alkyl, F, CL, Br, I, NO₂, CN, -NCS, -SCN; R³ is selected from the group consisting of: alkyl, aralkyl, alkaryl, polyalkylene oxide; and each of R¹³, R¹⁴, R¹⁵ and R¹⁶ is independently a polyalkylene oxide.
14.The perylene compound of clause 13, wherein each of A and B is H; and each of R¹³, R¹⁴, R¹⁵ and R¹⁶ comprises (CₐH₂ₐO)ₘ(C_{b}H_{2b}O)ₙOCH₃, where a and b are different and either 2 or 3, m is at least 3, and n is 1 - 31.wherein each of R¹³ and R¹⁴ comprises (CₐH₂ₐO)ₘ(C_{b}H_{2b}O)ₙCH₃, where a and b are different and either 2 or 3, m is at least 3, and n is 1 - 31.
15. An ink or a coating composition comprising the compound of clause 13.
16. The composition of clause 15, wherein said compound is present in an amount from about 0.01 to about 20% by weight.
17.The composition of clause 15 being a water-based ink jet ink.
18. The composition of clause 15 being a solvent based ink jet ink.
19. The composition of clause 15 being a flexo ink.
20. The composition of clause 15 being an energy curable ink.
21. The composition of clause 15 being an offset ink.
22.The composition of clause 15 being a coating composition for security applications.
23. An article coated with the composition of clause 15.
24. A method of tagging an article with a fluorescent taggant comprising coating said article with the coating composition of clause 15.
25. A triarylmethane compound having one of the following chemical formulae III or IV: 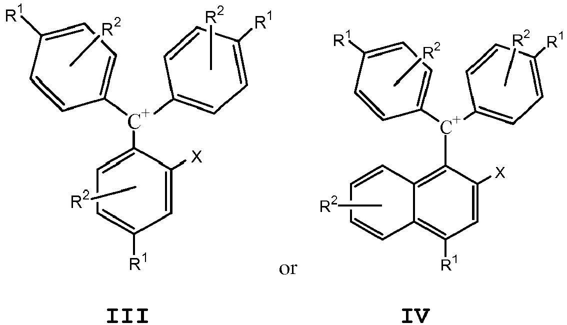 wherein X is H or CO₂⁻ or CO₂H [with a counterion Z⁻]; R¹ is selected from the group consisting of H, NR³₂, OR⁴, alkyl, aryl and substituted aryl; R² is selected from the group consisting of H, SO₃H, SO₃⁻(1/n)Mⁿ⁺, CO₂H and CO₂-(1/n)Mⁿ⁺; each of R³, R⁴ is independently selected from the group consisting of alkyl, aralkyl, alkaryl and polyalkylene oxide; Z is selected from the group consisting of halide, tosylate, brosylate, carboxylate, sulphate and phosphate; M is a cation; and n is integer from 1 to 4.
26. An ink or a coating composition comprising the compound of clause 25.
27. The composition of clause 26, wherein said compound is present in an amount from about 0.01 to about 20% by weight.
28.The composition of clause 26 being a water-based ink jet ink.
29. The composition of clause 26 being a solvent based ink jet ink.
30. The composition of clause 26 being a flexo ink.
31. The composition of clause 26 being an energy curable ink.
32. The composition of clause 26 being an offset ink.
33.The composition of clause 26 being a coating composition for security applications.
34. An article coated with the composition of clause 26.
35. A method of tagging an article with a fluorescent taggant comprising coating said article with the coating composition of clause 26.
36. A xanthene or thioxanthene compound having one of the following chemical formula V: 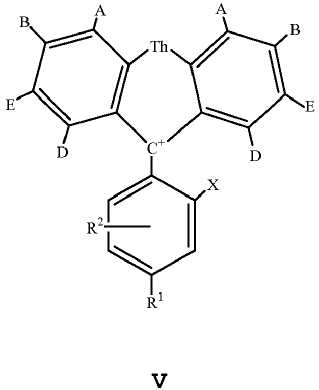 wherein Th is 0 or S; R¹ is selected from the group consisting of H, NR³₂, OR⁴, alkyl, aryl and substituted aryl; R² is selected from the group consisting of H, SO₃H, SO₃⁻(1/n)Mⁿ⁺, CO₂H and CO₂-(1/n)Mⁿ⁺; and each of A, B, E and D is independently selected from the group consisting of H, OH, OR⁴, NR³₂, F, CL, Br, I, NO₂, CN, ⁻ NCS and -SCN.
37. An ink or a coating composition comprising the compound of clause 36.
38. The composition of clause 37, wherein said compound is present in an amount from about 0.01 to about 20% by weight.
39.The composition of clause 37 being a water-based ink jet ink.
40. The composition of clause 37 being a solvent based ink jet ink.
41. The composition of clause 37 being a flexo ink.
42. The composition of clause 37 being an energy curable ink.
43. The composition of clause 37 being an offset ink.
44.The composition of clause 37 being a coating composition for security applications.
45. An article coated with the composition of clause 37.
46. A method of tagging an article with a fluorescent taggant comprising coating said article with the coating composition of clause 37.
47.A cyanine compound having the following chemical formula VI: 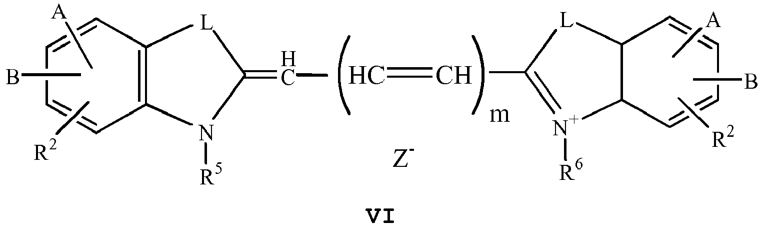 wherein L is selected from the group consisting of 0, S and N-R⁷; A and B is independently selected from the group consisting of H, OH, OR⁴, NR³₂, F, CL, Br, I, NO₂, CN, -NCS and ⁻SCN; R² is selected from the group consisting of H, SO₃H, SO₃⁻(1/n)Mⁿ⁺, CO₂H and CO₂-(1/n)Mⁿ⁺; m is integer from 0 to 3; Z is selected from the group consisting of halide, tosylate, brosylate, carboxylate, sulphate and phosphate; and each of R⁵, R⁶, R⁷ is independently selected from the group consisting of alkyl, carboxyalkyl, alkylsulfonate, alklyaryl and polyalkylene oxide.
48. An ink or a coating composition comprising the compound of clause 47.
49. The composition of clause 48, wherein said compound is present in an amount from about 0.01 to about 20% by weight.
50.The composition of clause 48 being a water-based ink jet ink.
51. The composition of clause 48 being a solvent based ink jet ink.
52. The composition of clause 48 being a flexo ink.
53. The composition of clause 48 being an energy curable ink.
54. The composition of clause 48 being an offset ink.
55.The composition of clause 48 being a coating composition for security applications.
56. An article coated with the composition of clause 48.
57. A method of tagging an article with a fluorescent taggant comprising coating said article with the coating composition of clause 48.
58.A diazopyrrole compound having the following chemical formula VII: 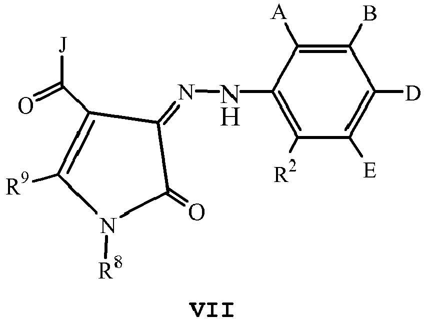 wherein each of A, B, E and D is independently selected from the group consisting of H, OH, OR⁴, N(R³)₂, F, CL, Br, I, NO₂, CN, -NCS, -SCN and substituted aryl; R² is selected from the group consisting of H, SO₃H, SO₃⁻(1/n)Mⁿ⁺, CO₂H and CO₂-(1/n)Mⁿ⁺; each of R³ and R⁴ is independently selected from the group consisting of alkyl, aralkyl, alkaryl and polyalkylene oxide; R⁸ is selected from the group consisting of H, alkyl, aryl, substituted aryl, aralkyl, alkaryl and imidino; R⁹ is selected from the group consisting of H, alkyl, -N(R³)₂, -OR⁴, aryl and substituted aryl; and J is NH-polyalkyene oxide or 0-polyalkylene oxide.
59. The compound of clause 58 having the following chemical formula XXV: 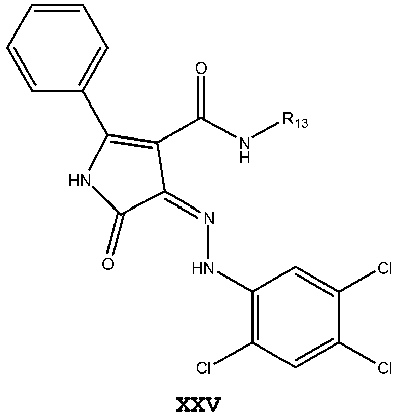 wherein R¹³ comprises (CₐH₂ₐO)ₘ(C_{b}H_{2b}O)ₙCH₃, where a and b are different and either 2 or 3, m is at least 3, and n is 1 - 31.
60. An ink or a coating composition comprising the compound of clause 58.
61. The composition of clause 60, wherein said compound is present in an amount from about 0.01 to about 20% by weight.
62.The composition of clause 60 being a water-based ink jet ink.
63. The composition of clause 60 being a solvent based ink jet ink.
64. The composition of clause 60 being a flexo ink.
65. The composition of clause 60 being an energy curable ink.
66. The composition of clause 60 being an offset ink.
67.The composition of clause 60 being a coating composition for security applications.
68. An article coated with the composition of clause 60.
69. A method of tagging an article with a fluorescent taggant comprising coating said article with the coating composition of clause 60.
70. A phthalocyanine compound having the following chemical formula IX: 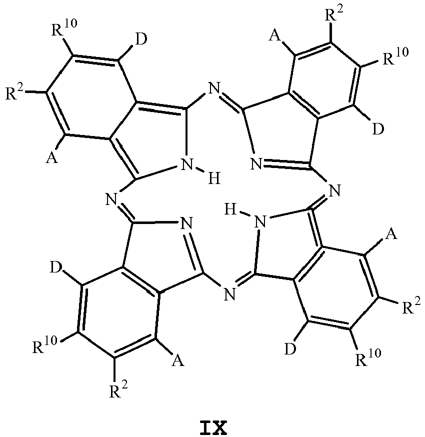 wherein each of A and D is independently selected from the group consisting of H, OH, OR⁴, N(R³)₂, F, CL, Br, I, NO₂, CN, -NCS and ⁻SCN; R² is selected from the group consisting of H, SO₃H, SO₃-(1/n)Mⁿ⁺, CO₂H and CO₂-(1/n)Mⁿ⁺; and R¹⁰ is selected from the group consisting of R², A, N(R³)₂, OR⁴ and SR⁴.
71. An ink or a coating composition comprising the compound of clause 70.
72. The composition of clause 71, wherein said compound is present in an amount from about 0.01 to about 20% by weight.
73.The composition of clause 71 being a water-based ink jet ink.
74. The composition of clause 71 being a solvent based ink jet ink.
75. The composition of clause 71 being a flexo ink.
76. The composition of clause 71 being an energy curable ink.
77. The composition of clause 71 being an offset ink.
78.The composition of clause 71 being a coating composition for security applications.
79. An article coated with the composition of clause 71.
80. A method of tagging an article with a fluorescent taggant comprising coating said article with the coating composition of clause 71.
81. A phthalocyanine compound having the following chemical formula **X:** 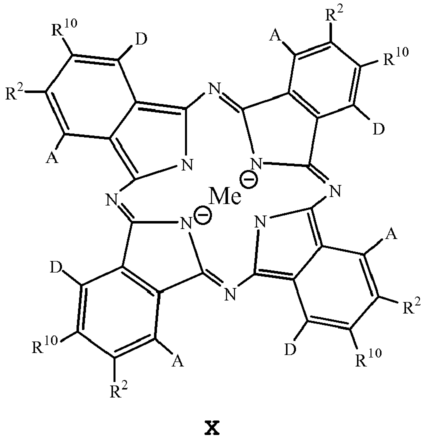 wherein Me is MXₙ(O)ₚ²⁺, M is a cation derived from a metallic or semimetallic element, X is a halide or alkoxide, 0 is an oxygen atom, n is an integer from 0 to 3, and p is an integer from 0 to 1, each of A and D is independently selected from the group consisting of H, OH, OR⁴, N(R³)₂, F, CL, Br, I, NO₂, CN, ⁻NCS and ⁻SCN; R² is selected from the group consisting of H, SO₃H, SO₃⁻(1/n)Mⁿ⁺, CO₂H and CO₂-(1/n)Mⁿ⁺; and R¹⁰ is selected from the group consisting of R², A, N(R³)₂, OR⁴ and SR⁴.
82. The compound of clause 81, wherein X is selected from the group consisting of chloride, methoxide and ethoxide.
83. The compound of clause 81, wherein M is selected from the group consisting of Be²⁺, Mg²⁺, Ca²⁺, ScX²⁺, TiX₂²⁺, TiO²⁺, VOX²⁺, CrX²⁺ , Mn²⁺, AlX²⁺, Zn²⁺, SiO²⁺ and SiX₂²⁺.
84. An ink or a coating composition comprising the compound of clause 81.
85. The composition of clause 84, wherein said compound is present in an amount from about 0.01 to about 20% by weight.
86.The composition of clause 84 being a water-based ink jet ink.
87. The composition of clause 84 being a solvent based ink jet ink.
88. The composition of clause 84 being a flexo ink.
89. The composition of clause 84 being an energy curable ink.
90. The composition of clause 84 being an offset ink.
91.The composition of clause 84 being a coating composition for security applications.
92. An article coated with the composition of clause 84.
93. A method of tagging an article with a fluorescent taggant comprising coating said article with the coating composition of clause 84.
94. A naphthalocyanine compound having the following chemical formula **XI:** 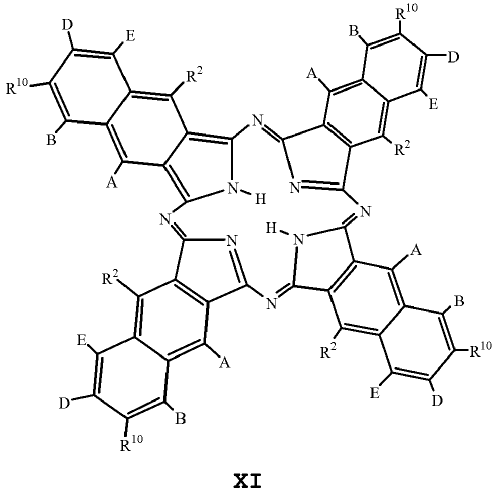 wherein each of A, D and E is independently selected from the group consisting of H, OH, OR⁴, NR³₂, F, CL, Br, I, NO₂, CN, - NCS and ⁻SCN; R² is selected from the group consisting of H, SO₃H, SO₃⁻(1/n)Mⁿ⁺, CO₂H and CO₂-(1/n)Mⁿ⁺; and R¹⁰ is selected from the group consisting of R², A, N(R³)₂, OR⁴ and SR⁴.
95. An ink or a coating composition comprising the compound of clause 94.
96. The composition of clause 95, wherein said compound is present in an amount from about 0.01 to about 20% by weight.
97.The composition of clause 95 being a water-based ink jet ink.
98. The composition of clause 95 being a solvent based ink jet ink.
99. The composition of clause 95 being a flexo ink.
100.The composition of clause 95 being an energy curable ink.
101. The composition of clause 95 being an offset ink.
102.The composition of clause 95 being a coating composition for security applications.
103. An article coated with the composition of clause 95.
104. A method of tagging an article with a fluorescent taggant comprising coating said article with the coating composition of clause 95.
105.A naphthalocyanine compound having the following chemical formula **XII:** 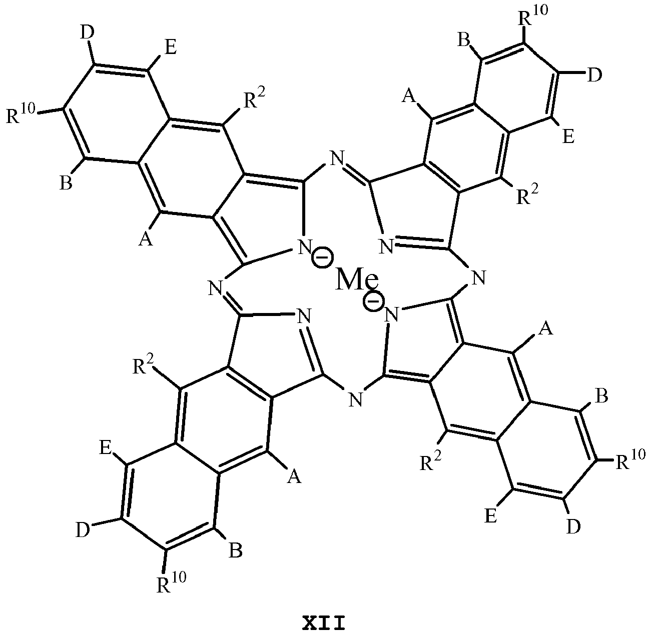 wherein Me is MXₙ(O)p²⁺, M is a cation derived from a metallic or semimetallic element, X is a halide or alkoxide, 0 is an oxygen atom, n is an integer from 0 to 3, and p is an integer from 0 to 1, each of A, D and E is independently H, OH, OR⁴, NR³₂, F, CL, Br, I, NO₂, CN, -NCS and ⁻SCN; R² is selected from the group consisting of H, SO₃H, SO₃⁻(1/n)Mⁿ⁺, CO₂H and CO₂-(1/n)Mⁿ⁺; and R¹⁰ is selected from the group consisting of R², A, N(R³)₂, OR⁴ and SR⁴.
106.The compound of clause 105, wherein X is selected from the group consisting of chloride, methoxide and ethoxide.
107. The compound of clause 105, wherein M is selected from the group consisting of Be²⁺, Mg²⁺, Ca²⁺, ScX²⁺, TiX₂²⁺, TiO²⁺, VOX²⁺, CrX²⁺ , Mn²⁺, AlX²⁺, Zn²⁺, SiO²⁺ and SiX₂²⁺.
108.An ink or a coating composition comprising the compound of clause 105.
109. The composition of clause 108, wherein said compound is present in an amount from about 0.01 to about 20% by weight.
110.The composition of clause 108 being a water-based ink jet ink.
111.The composition of clause 108 being a solvent based ink jet ink.
112. The composition of clause 108 being a flexo ink.
113.The composition of clause 108 being an energy curable ink.
114. The composition of clause 108 being an offset ink.
115.The composition of clause 108 being a coating composition for security applications.
116. An article coated with the composition of clause 108.
117. A method of tagging an article with a fluorescent taggant comprising coating said article with the coating composition of clause 108.
118.A coumarin or a carbostyril compound having the following chemical formula **XIII:** 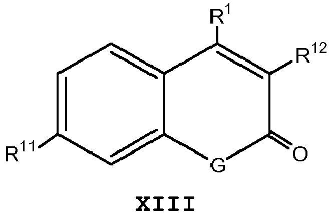 wherein R¹ is selected from the group consisting of H, N(R³)₂, OR⁴, alkyl, aryl and substituted aryl; R¹¹ is selected from the group consisting of: N(R³)₂, 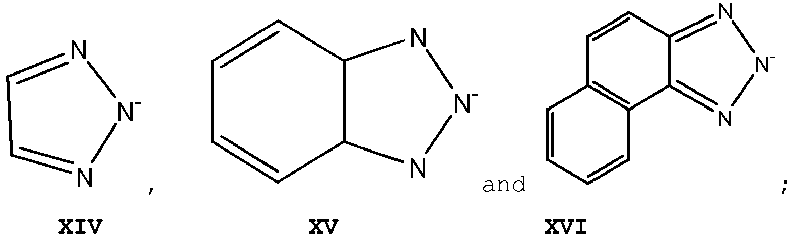 R¹² is or A; G is 0 or NR⁵; R⁵ is independently selected from the group consisting of alkyl, carboxyalkyl, alkylsulfonate, alkylaryl and polyalkylene oxide; and A is selected from the group consisting of H, OH, OR⁴, NR³₂, F, CL, Br, I, NO₂, CN, -NCS and -SCN.
119.An ink or a coating composition comprising the compound of clause 118.
120. The composition of clause 119, wherein said compound is present in an amount from about 0.01 to about 20% by weight.
121.The composition of clause 119 being a water-based ink jet ink.
122.The composition of clause 119 being a solvent based ink jet ink.
123. The composition of clause 119 being a flexo ink.
124.The composition of clause 119 being an energy curable ink.
125. The composition of clause 119 being an offset ink.
126.The composition of clause 119 being a coating composition for security applications.
127. An article coated with the composition of clause 119.
128.A method of tagging an article with a fluorescent taggant comprising coating said article with the coating composition of clause 119.
129. A dialkyl succinosuccinate (DMSS) compound having the following chemical formula XVII: 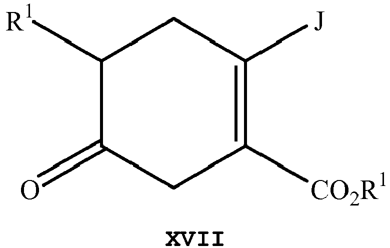 wherein R¹ is selected from the group consisting of H, NR³₂, OR⁴, alkyl, aryl and substituted aryl; and J is NH-polyalkyene oxide or O-polyalkylene oxide.
130. The dialkyl succinosuccinate compound of clause 129 having the following chemical formula XXVI: 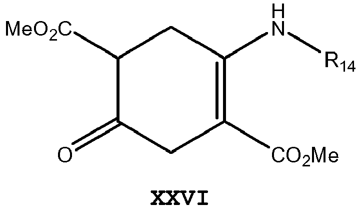 wherein R¹⁴ comprises (CₐH₂ₐO)ₘ(C_{b}H_{2b}O)ₙCH₃, where a and b are different and either 2 or 3, m is at least 3, and n is 1 - 31.
131.An ink or a coating composition comprising the compound of clause 129.
132. The composition of clause 131, wherein said compound is present in an amount from about 0.01 to about 20% by weight.
133.The composition of clause 131 being a water-based ink jet ink.
134.The composition of clause 131 being a solvent based ink jet ink.
135. The composition of clause 131 being a flexo ink.
136.The composition of clause 131 being an energy curable ink.
137. The composition of clause 131 being an offset ink.
138.The composition of clause 131 being a coating composition for security applications.
139. An article coated with the composition of clause 131.
140.A method of tagging an article with a fluorescent taggant comprising coating said article with the coating composition of clause 131.
141.A naphthaloperylene compound having the following chemical formula **XIX:** 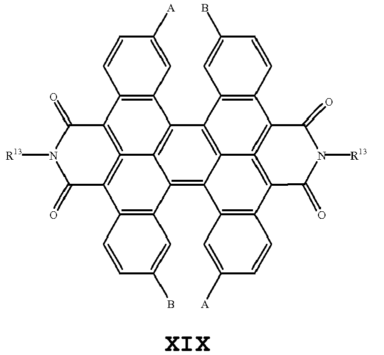 wherein each of A and B is independently H, OH, OR⁴, NR³₂, F, CL, Br, I, NO₂, CN, ⁻NCS and ⁻SCN; and R¹³ is polyalkylene oxide.
142. The naphthaloperylene compound of clause 141, wherein R¹³ comprises (CₐH₂ₐO)ₘ(C_{b}H_{2b}O)ₙCH₃, where a and b are different and either 2 or 3, m is at least 3, and n is 1 - 31.
143.An ink or a coating composition comprising the compound of clause 141.
144. The composition of clause 143, wherein said compound is present in an amount from about 0.01 to about 20% by weight.
145.The composition of clause 143 being a water-based ink jet ink.
146.The composition of clause 143 being a solvent based ink jet ink.
147. The composition of clause 143 being a flexo ink.
148.The composition of clause 143 being an energy curable ink.
149. The composition of clause 143 being an offset ink.
150.The composition of clause 143 being a coating composition for security applications.
151. An article coated with the composition of clause 143.
152.A method of tagging an article with a fluorescent taggant comprising coating said article with the coating composition of clause 143.
153.A Lanthanide compound having the following chemical formula **XX:** 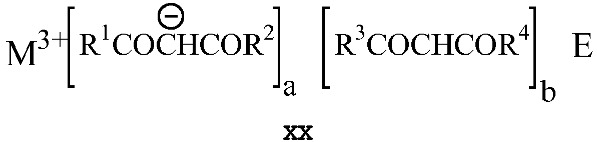 wherein M is a lanthanide; each of R1, R2 and R3 is independently selected from the group consisting of alkyl, alkaryl, aralkyl, cyano, haloalkyl, haloaryl, aryl, carboxy aryl, aryl sulfonate, heterocyclic and R⁴; R⁴ is selected from the group consisting of -NH [polyalkylene oxide], -0 [polyalkylene oxide],
   and 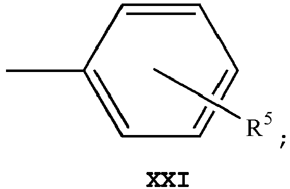 R⁵ is selected from the group consisting of -CONH [polyalkylene oxide], -CO₂ [polyalkylene oxide], -SO₃ [polyalkylene oxide] and -SO₂NO [polyalkylene oxide]; a is an integer from 0-2; b is an integer from 1-3; and E is 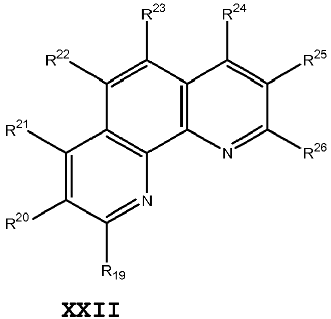 where R19 through R26 are each independently H, alkyl, alkoxy, halide, sulfonic acid.
154.The compound of clause 153, wherein M is selected from the group consisting of Eu, Tb, Sm and Dy.
155.An ink or a coating composition comprising the compound of clause 153.
156. The composition of clause 155, wherein said compound is present in an amount from about 0.01 to about 20% by weight.
157.The composition of clause 155 being a water-based ink jet ink.
158.The composition of clause 155 being a solvent based ink jet ink.
159. The composition of clause 155 being a flexo ink.
160.The composition of clause 155 being an energy curable ink.
161. The composition of clause 155 being an offset ink.
162.The composition of clause 155 being a coating composition for security applications.
163. An article coated with the composition of clause 155.
164.A method of tagging an article with a fluorescent taggant comprising coating said article with the coating composition of clause 155.
165.A Lanthanide compound having the following chemical formula **XXIII:** 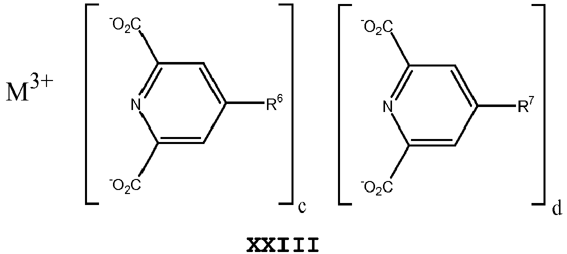 wherein M is a lanthanide; c is an integer from 0-2; d is an integer from 1-3; R⁶ is selected from the group consisting of H, alkyl, alkaryl, sulphonic acid, carboxylic acid, halide, alkoxy, sulphonamide, carboxamide and R⁷; and R⁷ is selected from the group consisting of CONH [polyalkylene oxide], CO₂ [polyalkylene oxide], SO₂NH [polyalkylene oxide] and SO₃ [polyalkylene oxide].
166.The compound of clause 165, wherein M is selected from the group consisting of Eu, Tb, Sm and Dy.
167.An ink or a coating composition comprising the compound of clause 165.
168. The composition of clause 167, wherein said compound is present in an amount from about 0.01 to about 20% by weight.
169.The composition of clause 167 being a water-based ink jet ink.
170.The composition of clause 167 being a solvent based ink jet ink.
171. The composition of clause 167 being a flexo ink.
172.The composition of clause 167 being an energy curable ink.
173. The composition of clause 167 being an offset ink.
174.The composition of clause 167 being a coating composition for security applications.
175. An article coated with the composition of clause 167.
176.A method of tagging an article with a fluorescent taggant comprising coating said article with the coating composition of clause 167.
177.A method of tagging an article with a fluorescent taggant comprising coating said article with a coating composition containing a compound having the following chemical formula **XXIV:**

   K-T_{w} XXIV

   wherein K is a fluorescent moiety; T is a pendant group comprising a polyalkylene oxide residue; and w is a number greater than zero.
178.The method of clause 177, wherein said fluorescent moiety is selected from the group consisting of perylene, xanthene, thioxanthene, cyanine, diazopyrrole, phthalocyanine, naphthalocyanine, coumarin, carbostyril, dialkylsuccinosuccinate, naphthaloperylene, lanthanide and derivatives thereof.
179. An article coated or tagged by the method of clause 177.

## Claims

1. A phthalocyanine compound having the following chemical formula **IX:** 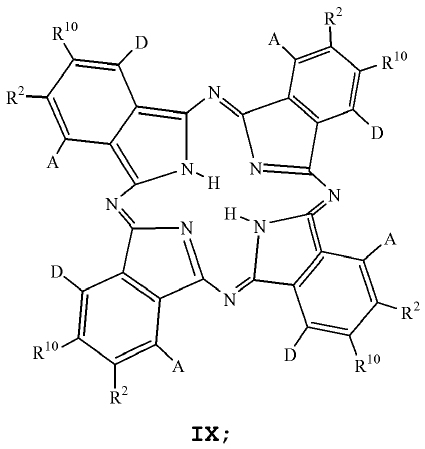 a phthalocyanine compound having the following chemical formula **X:** 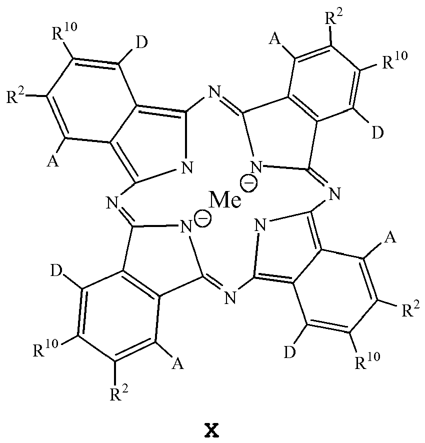 in which Me is MXₙ(O)ₚ²⁺, M is a cation derived from a metallic or semimetallic element, X is a halide or alkoxide, O is an oxygen atom, n is an integer from 0 to 3, and p is an integer from 0 to 1;
a naphthalocyanine compound having the following chemical formula **XI:** 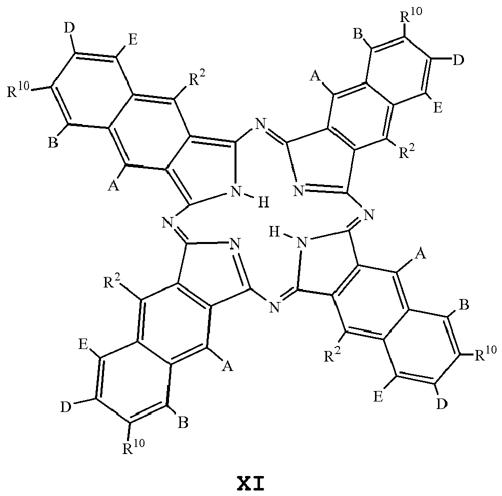 in which E is independently selected from the group consisting of H, OH, OR⁴, NR³₂, F, CL, Br, I, NO₂, CN, -NCS and -SCN;
or
a naphthalocyanine compound having the following chemical formula **XII:** 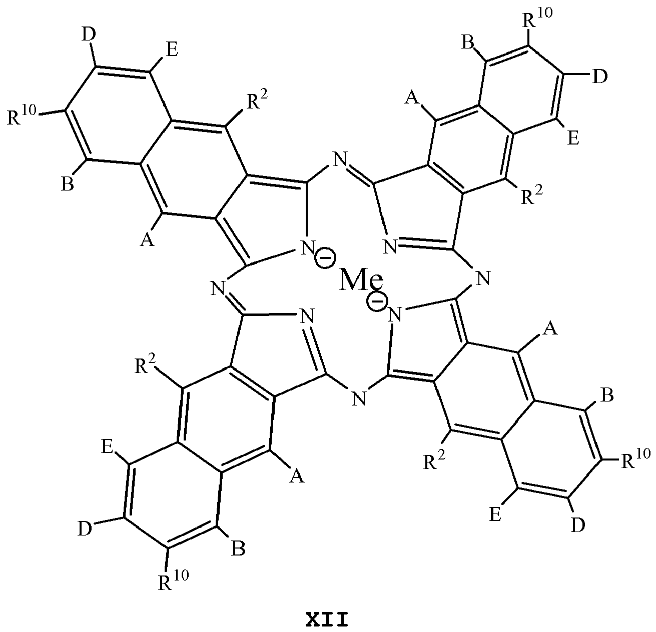 in which Me is MXₙ(O)ₚ²⁺, M is a cation derived from a metallic or semimetallic element, X is a halide or alkoxide, O is an oxygen atom, n is an integer from 0 to 3, and p is an integer from 0 to 1, and E is independently H, OH, OR⁴, NR³₂, F, CL, Br, I, NO₂, CN, -NCS and ⁻SCN;
wherein in each of the phthalocyanine compounds of chemical formulae **IX** and **X,** and the naphthalocyanine compounds of chemical formula **XI** and **XII,** each of A and D is independently selected from the group consisting of H, OH, OR⁴, N(R³)₂, F, CL, Br, I, NO₂, CN, -NCS and -SCN; R² is selected from the group consisting of H, SO₃H, SO₃⁻(¹/n)Mⁿ⁺, CO₂H and CO₂-(¹/n)Mⁿ⁺; and R¹⁰ is selected from the group consisting of R², A, N(R³)₂, OR⁴ and SR⁴.

2. The phthalocyanine compound of formula **X** or the naphthalocyanine compound of formula **XII** of claim 1, wherein X is selected from the group consisting of chloride, methoxide and ethoxide.

3. The phthalocyanine compound of formula **X** or the naphthalocyanine compound of formula **XII** of claim 1 or claim 2, wherein M is selected from the group consisting of Be²⁺, Mg²⁺, Ca²⁺, ScX²⁺, TiX₂²⁺, TiO²⁺, VOX²⁺, CrX²⁺, Mn²⁺, AlX²⁺, Zn²⁺, SiO²⁺ and SiX₂²⁺.

4. An ink or a coating composition comprising the compound of any one of claims 1 to 3.

5. The composition of claim 4, wherein said compound is present in an amount from about 0.01 to about 20% by weight.

6. The composition of claim 4 or claim 5 being a water-based ink jet ink or a solvent based ink jet ink.

7. The composition of claim 4 or 5 being a flexo ink, an energy curable ink or an offset ink.

8. The composition of any one of claims 4 to 7 being a coating composition for security applications.

9. An article coated with the composition of any one of claims 4 to 8.

10. A method of tagging an article with a fluorescent taggant comprising coating said article with the coating composition of any one of claims 4 to 9.

11. A method of tagging an article with a fluorescent taggant comprising coating said article with a coating composition containing a compound having the following chemical formula **XXIV:** 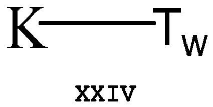 wherein K is a fluorescent moiety selected from phthalocyanine, naphthalocyanine and derivatives thereof; T is a pendant group comprising a polyalkylene oxide residue; and w is a number greater than zero.

12. The method of claim 11, wherein K is a fluorescent moiety selected from the phthalocyanine compounds of chemical formulae **IX** and **X** as defined in any one of claims 1 to 3, the naphthalocyanine compounds of chemical formula **XI** and **XII** as defined in any one of claims 1 to 3, and derivatives thereof.

13. An article coated or tagged by the method of claim 11 or claim 12.
